# EUROPÄISCHE PATENTSCHRIFT

(11) **EP 2 709 958 B1**
(45) Veröffentlichungstag und Bekanntmachung des Hinweises auf die Patenterteilung: **20.12.2017**
(21) Anmeldenummer: 12726019.8
(22) Anmeldetag: 16.05.2012
(51) Int. Cl.: C02F 1/32

(54) **STEUERUNG FÜR EINE UV-DESINFEKTIONSANLAGE MIT BREITBAND-UV-STRAHLERN**
SYSTEM FOR CONTROLLING AN UV DISINFECTION INSTALLATION USING A UV BROADBAND RADIATION
SYSTÈME DE RÉGULATION D'UNE INSTALLATION DE DÉSINFECTION UV UTILISSANT UNE RAYONNEMENTS UV A LARGE BANDE

(30) Priorität: 20.05.2011 DE 102011102687
(43) Veröffentlichungstag der Anmeldung: 26.03.2014
(73) Patentinhaber: Xylem IP Holdings LLC, White Plains, NY 10604 (US)
(72) Erfinder: BOKERMANN, Christian, 32130 Enger (DE); KÄMMERER, Sven, 32107 Bad Salzuflen (DE)
(74) Vertreter: Lenzing Gerber Stute
(86) Internationale Anmeldenummer: PCT/EP2012/002109
(87) Internationale Veröffentlichungsnummer: WO 2012/159719

(56) Entgegenhaltungen:
- WO-A2-01/92839
- US-A- 6 057 917
- US-A1- 2004 061 069
- US-B1- 6 429 438

## Beschreibung

Die vorliegende Erfindung betrifft eine Vorrichtung zur Wasserdesinfektion, die einen Breitband-UV-Strahler und einen UV-Sensor enthält, sowie ein Verfahren zur Steuerung von UV-Desinfektionsanlagen, in denen Breitband-UV-Strahler eingesetzt werden. Mit dem erfindungsgemäßen Verfahren soll eine möglichst präzise Steuerung der Leistung der Desinfektionsanlage erzielt werden, um einerseits die erforderliche Desinfektionsleistung sicher zu erbringen und andererseits einen unnötig hohen Energieverbrauch vermeiden zu können.

### Technischer Hintergrund

Die keimtötende, desinfizierende Wirkung von UV-Strahlung ist seit langem bekannt. UV-Strahler werden seit vielen Jahrzehnten für Desinfektion von Trinkwasser und Abwasser, in Klimaanlagen, Sümpfen und zur Desinfektion von Arbeitsbereichen in biologischen Laboratorien eingesetzt. Bei der Desinfektion von Wasser wird UV-Strahlung erzeugt und in das Wasser abgegeben, so dass Keime (Viren, Bakterien, Einzeller) erreicht werden. Die keimtötende Wirkung der UV-Strahlung ist dabei abhängig von der Wellenlänge und von der Art der Mikroorganismen, die inaktiviert werden sollen.

Als ultraviolette Strahlung oder ultraviolettes Licht bezeichnet man den Wellenlängenbereich der elektromagnetischen Strahlung, der zwischen dem sichtbaren Licht und der Röntgenstrahlung liegt, also etwa den Wellenlängenbereich von 400 nm - 100 nm. Hierbei wird das gesamte UV-Spektrum üblicherweise in 4 Bereiche aufgeteilt, nämlich das UV-A (315 nm - 400nm), UV-B (280 nm - 315nm), UV-C (200 nm - 280 nm) und Vakuum-UV oder V-UV (100 nm - 200 nm). Die keimtötende, desinfizierende Wirkung von UV-Strahlung wird hauptsächlich mit den Wellenlängenbereichen des UV-B und UV-C erzielt. Die keimtötende Wirkung von UV-A ist im Vergleich zu UV-B und UV-C relativ gering.

Nahezu alle Wasserentkeimungsanlagen werden mit UV-Strahlern betrieben, die als Gasentladungslampen mit einem Quecksilberanteil in der Gasfüllung ausgebildet sind. Quecksilber produziert unter anderem eine dominante Emissionslinie bei 254 nm, die nahe bei einem Maximum der wellenlängenabhängigen Wirksamkeit von UV-Licht zur Desinfektion von Mikroorganismen liegt. Die Abhängigkeit der Wirksamkeit von der Wellenlänge weist bei den meisten Mikroorganismen ein lokales Maximum bei 260 nm auf, d.h. eine bestimmte Dosis an UV-Strahlung dieser Wellenlänge ist besonders wirksam. In Richtung der kürzeren Wellenlängen fällt die Wirksamkeit zunächst bis 240 nm ab und steigt dann wieder an. Der Bereich zwischen 240 nm und 200 nm ist ebenfalls mit gutem Wirkungsgrad für die Desinfektion von Mikroorganismen geeignet.

Bei den Strahlern kommen hauptsächlich zwei Bauarten zum Einsatz, nämlich einmal sogenannte Niederdruckstrahler, die mit einem Gasdruck von weniger als etwa 0,1 mbar betrieben werden. Diese Niederdruckstrahler weisen ein sehr schmalbandiges Linienspektrum auf und emittieren im genannten Wellenlängenbereich praktisch ausschließlich UV-C-Strahlung der Wellenlänge 254 nm. Sie zeichnen sich durch einen sehr hohen elektrischen Wirkungsgrad aus, da rund 40% der gesamten aufgenommenen elektrischen Leistung in Strahlungsleistung der genannten Wellenlänge umgesetzt wird. Nachteilig ist bei Niederdruckstrahlern, dass die absolute Strahlungsleistung im Verhältnis zur Baugröße relativ gering ist, so dass für eine Desinfektionsanlage mit großem Durchsatz an Wasser eine Vielzahl von Strahlern eingesetzt werden muss. Die Anlagen werden entsprechend aufwändig. Vorteilhaft ist wiederum, dass zur Regelung der Strahler lediglich die Strahlungsleistung bei 254 nm überwacht werden muss, da andere Komponenten nur einen unwesentlichen Anteil an der Desinfektionsleistung haben und die wirksame Linie deshalb direkt zur Steuerung der Anlage genützt werden kann.

Das Dokument DE 20 2004 012 686 U1 beschreibt eine Desinfektionsvorrichtung mit einem UV-Strahler, der in einem Kolben angeordnet ist und dessen Emission mit einem nicht näher beschriebenen Sensor überwacht wird. Bei Abweichung von einem Sollwert soll ein Signal abgegeben werden, das auf die erforderliche Reinigung hinweist.

Aus dem Dokument DE 10 2008 051 239 A1 ist eine Desinfektionsanlage mit UV-Strahlern bekannt, die mit zwei Sensoren überwacht werden. Eine Alterung und eine mögliche Dämpfung der gesamten Strahlungsleistung werden mittels einer unterschiedlichen geometrischen Anordnung der Sensoren, z.B. durch einen unterschiedlichen Abstand oder einen unterschiedlichen Erfassungswinkel ermittelt. Es wird hier im wesentlichen das Lambert-Beer'sche Gesetz ausgenutzt, um eine Trübung der Umhüllung der Strahler aufgrund von Ablagerungen zu ermitteln. Einen Hinweis auf die spektrale Empfindlichkeit der UV-Sensoren enthält dieses Dokument nicht. Es ist auch nicht dargelegt, dass die beiden UV-Sensoren unterschiedliche spektrale Empfindlichkeiten aufweisen können sollen.

Andere Anlagen zur Wasserdesinfektion nutzen sogenannte Mitteldruckstrahler. Der interne Druck dieser Strahler beträgt etwa 0,1 - 10 bar. Die Strahler werden bei höheren Temperaturen betrieben und weisen bei wesentlich kompakteren Abmessungen eine um ein vielfaches höhere elektrische Leistungsaufnahme und eine entsprechend höhere UV-Strahlungsleistung auf. Durch die höheren Temperaturen und den höheren Druck innerhalb des Strahlers werden auch andere UV-C-Linien und eine Kontinuumsstrahlung angeregt, die in dem Bereich zwischen 240 nm und 200 nm liegen. Dieser Bereich ist, wie oben gesagt wurde, für die Desinfektionsleistung ebenfalls relevant, da die Wirkung einer gegebenen UV-Dosis auf Mikroorganismen in diesem Wellenlängenbereich ebenfalls hoch ist.

Das Dokument US 6,429,438 B1 offenbart eine Vorrichtung zur Messung von UV-Strahlung mit zwei Sensoren, deren Signale verglichen werden. Die spektrale Empfindlichkeit der beiden Sensoren soll von 190 nm bis 310 nm gehen. Ein Empfindlichkeitsmaximum ist nicht offenbart.

Das Dokument US 6,057,917 A offenbart einen Sensor für die Messung von UV-Strahlungsintensitäten im Wellenlängenbereich von 200 nm bis 400 nm. Ein Empfindlichkeitsmaximum ist nicht offenbart.

Das Dokument WO 01/92839 A2 offenbart einen Sensor für die Messung von UV-Strahlungsintensitäten, wobei die größte Empfindlichkeit in einem Wellenlängenbereich zwischen 240 nm und 320 nm liegen soll und ein Empfindlichkeitsmaximum bei 260 nm bevorzugt ist.

Ähnlich wie bei der Überwachung und Regelung der Strahlungsleistung von Niederdruckstrahlern erfolgt bislang auch die Überwachung von Mitteldruckstrahlern. Hierzu werden UV-Sensoren eingesetzt, die einen Teil des Spektrums oder das gesamte Spektrum erfassen. Die Leistung des Mitteldruckstrahlers wird dann so geregelt, dass die vom Sensor erfasste Gesamtemission den Anforderungen und Vorgaben entspricht. Es wird im Stand der Technik also ein Spektrum erfasst, das von Linien und einem Kontinuum mit Wellenlängen von mehr als 240 nm dominiert wird. Ein Ultraviolettsensor für eine solche Überwachung ist beispielsweise in dem Dokument US 2004/0200975 A1 beschrieben. Hier wird ein SiC-Sensor offenbart, der ein Empfindlichkeitsmaximum etwa bei 260 nm aufweist.

Es hat sich gezeigt, dass diese Überwachung von Mitteldruckstrahlern für eine Regelung und Überwachung hinsichtlich der Desinfektionsleistung des UV-Strahlers nur beschränkt geeignet ist. Es ist deshalb Aufgabe der vorliegenden Erfindung, eine Vorrichtung zur Überwachung und Regelung der Strahlungsleistung von Mitteldruckstrahlern in Wasserentkeimungsanlagen zu schaffen, mit der die biologisch wirksame UV-C-Strahlung derart geregelt werden kann, dass eine vorgegebene Entkeimungsleistung zuverlässig erreicht wird und dennoch ein unnötig hoher Einsatz elektrischer Energie vermieden werden kann.

Diese Aufgabe wird von einer Vorrichtung mit den Merkmalen des Anspruchs 1 und von einem Verfahren mit den Merkmalen des Anspruchs 4 gelöst.

Weil die Vorrichtung einen ersten UV-Sensor aufweist, der sein Empfindlichkeitsmaximum für UV-Strahlung in einem Bereich zwischen 200 und 230 nm aufweist, und bevorzugt ein Maximum bei 220 nm aufweist, kann mit diesem ersten UV-Sensor der biologisch besonders wirksame Spektralbereich von 200 - 240 nm erfasst werden und bereits geringe Schwankungen der abgegebenen Intensität in diesen Wellenlängenbereich kompensiert werden, die sich in der Gesamtintensität der Strahlung im gesamten UV-Bereich und im UV-C-Bereich nicht signifikant ändern. Vorteilhaft werden zwei Sensoren eingesetzt, wobei ein zweiter UV-Sensor den Spektralbereich mit Wellenlängen oberhalb von 240 nm erfasst. Auf diese Weise kann das Verhältnis zwischen der Strahlungsleistung im Bereich 200 nm bis 240 nm und der Strahlungsleistung im Bereich 240 nm bis 300 nm verglichen werden. Dieses Verhältnis ist ein Indiz für den technischen Zustand des Strahlers, beispielsweise hinsichtlich der Betriebsbedingungen und der Alterung.

Alternativ kann auch ein Sensor eingesetzt werden, dessen spektrale Empfindlichkeit zwischen den beiden genannten Wellenlängenbereichen umschaltbar ist. Ein solcher Sensor würde dann den ersten UV-Sensor und den zweiten UV-Sensor in Form von zwei separaten UV-sensitiven Elementen umfassen, die in einem Sensorgehäuse integriert sind.

Es kann aber auch vorgesehen sein, einen nach Art eines Spektrometers auflösenden Sensor einzusetzen, mit dem der kurzwellige Teil des UV-C-Bereichs zwischen 200 nm und 240 nm gemessen wird. Der Wellenlängenbereich von 200 nm - 240 nm aus dem aufgelösten Spektrum wird ausgewertet und zur Regelung des UV-Strahlers und / oder zur Berechnung der tatsächlichen Desinfektionsleistung der Anlage genutzt.

Der erste UV-Sensor für den Bereich von 200 nm - 240 nm ist vorzugsweise ein Halbleitersensor, der mit Filtermitteln versehen ist, die den Wellenlängenbereich zwischen 200 nm und 240 nm durchlassen und andere Wellenlängen, insbesondere den Bereich mit Wellenlängen > 240 nm sperren.

Weil bei dem erfindungsgemäßen Verfahren zur Überwachung und Steuerung einer Wasserdesinfektionsanlagen mit wenigstens einem in einem Kanal angeordneten Breitband-UV-Strahler beispielsweise von der Bauart eines Quecksilbermitteldruckstrahlers oder eines Excimerstrahlers, wobei wenigstens ein erster UV-Sensor vorgesehen ist, der in dem Wasser in einem Abstand von dem Breitband-UV-Strahler angeordnet ist, und der erste UV-Sensor mit einer Regeleinheit verbunden ist, die zur Regelung der Leistung des Breitband-UV-Strahlers oder des Volumenstroms an Wasser durch den Kanal eingerichtet ist,
- der erste UV-Sensor (103) ein Empfindlichkeitsmaximum im Bereich zwischen 200 nm und 230 nm aufweist,
- ein Signal des ersten UV-Sensors (103) im Betrieb ausgewertet wird,
- aus dem Signal des ersten UV-Sensors (103) eine UV-Dosis in dem Wellenlängenbereich von 200 nm bis 240 nm berechnet wird, und
- die am Ort des ersten UV-Sensors (103) ermittelte UV-Dosis als Maß für die Berechnung Desinfektionsleistung der Wasserdesinfektionsanlage verwendete wird,
kann jederzeit die erzielte Desinfektionsleistung genau festgestellt und ggf. nachgeregelt werden.

Wenn weiter vorgesehen ist, dass für die Berechnung der Desinfektionsleistung ein UV-Wirkungsspektrum eines vorbestimmten Mikroorganismus zugrunde gelegt wird, kann bei genauer bekannter mikrobieller Belastung des Wassers oder Abwassers die erforderliche Inaktivierungsrate für diesem Mikroorganismus gemessen und ggf. eingeregelt werden.

Wenn weiter für die Berechnung der Desinfektionsleistung der Mikroorganismus aus einer Gruppe auswählbar ist, die Viren, Bakterien und Einzeller umfasst, kann auf verschiedene anfallende Belastungen reagiert werden, z.B. bei der Nachbehandlung des Ablaufs einer Kläranlage.

Wenn ein zweiter UV-Sensor mit einem Empfindlichkeitsmaximum zwischen 240 nm und 300 nm vorgesehen ist, kann aus dem Verhältnis der Signale des ersten UV-Sensors und des zweiten UV-Sensors ein Messwert ermittelt wird, der die Alterung des Breitband-UV-Strahlers angibt.

Wenn schließlich in Abhängigkeit von dem Signal des Sensors die elektrische Leistung des Breitband-UV-Strahlers geregelt wird, können sowohl eine sich ändernde UV-Transmission des Wassers als auch eine beginnende Alterung des Strahlers ausgeglichen werden.

Nachfolgend wird ein Ausführungsbeispiel der vorliegenden Erfindung anhand der Zeichnung näher beschrieben. Die Zeichnung stellt auch allgemeinen technischen Hintergrund in Form von UV-Spektren verschiedener Strahler und Wirkungsdiagramme für die Entkeimungsleistung verschiedener Wellenlängen an verschiedenen Mikroorganismen dar. Im Einzelnen zeigen:
- Figur 1:: einen Vergleich zwischen dem Spektrum eines Niederdruckstrahlers und dem Spektrum eines Mitteldruckstrahlers (Stand der Technik);
- Figur 2:: die Abhängigkeit der Wirkung von UV-Strahlen auf verschiedene Mikroorganismen von der Wellenlänge (Stand der Technik);
- Figur 3:: beispielhafte Transmissionsspektren für verschiedene Quarzglassorten;
- Figur 4:: einige Absorptionsspektren von Wasserproben im Bereich 200 - 300nm (Stand der Technik aus: *USEPA (2006) Ultraviolet Disinfection Guidance Manual for the Long Term 2 Enhanced Surface Water Treatment Rule, EPA 815-R-06-007. Office of Water, Washington, DC.);*
- Figur 5:: Spektren von Mitteldruckstrahlern in Abhängigkeit von der Alterung der Strahler;
- Figur 6:: eine erfindungsgemäße Anordnung in einem schematischen Blockschaltbild; sowie
- Figur 7:: bevorzugte Empfindlichkeitskurven für die Sensoren aus Figur 6.

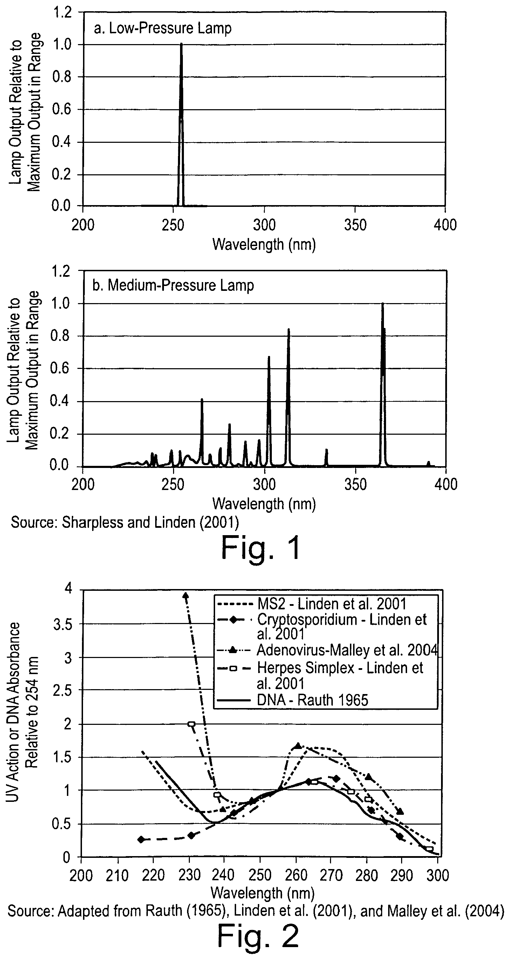

Die Figuren 1, 2 und 4 zeigen Abbildungen aus dem Stand der Technik, die zur Erläuterung des technischen Sachverhaltes zweckmäßig sind.

Die Figur 1 zeigt zwei verschiedene Spektren. Oben in Figur 1 ist die Emissionslinie eines Niederdruckstrahlers bei 254 nm dargestellt. Es ist sofort ersichtlich, dass eine Überwachung der UV-Emission bei dieser Wellenlänge geeignet ist, um die gesamte UV-Strahlungsleistung eines Niederdruckstrahlers auf einen bestimmten Sollwert zu regeln.

Die Figur 1 zeigt unten das Spektrum eines Mitteldruckstrahlers. Hier ist erkennbar, dass eine Vielzahl von Linien in dem Bereich zwischen 240 nm und etwa 370nm enthalten sind. Eine Regelung der Leistung eines solchen Mitteldruckstrahlers allein aufgrund eines UV-Sensors, der ein Empfindlichkeitsmaximum bei 260 nm aufweist, kann nur dann eine Regelung für die Gesamt-UV-Strahlungsleistung sein, wenn sich die relativen Intensitäten der verschiedenen Linien und des darunter liegenden Kontinuums nicht ändern.

In der Praxis zeigt sich jedoch, dass zum einen der Anteil an UV-Strahlung im Bereich von 200 - 240 nm von der Bauart des Mitteldruckstrahlers abhängig ist. Moderne Hochleistungsstrahler emittieren in diesem kurzwelligen Bereich nennenswerten Anteil Ihrer gesamten Strahlungsleistung, während ältere Strahler, die anders aufgebaut sind und betrieben werden, nur einen geringen Teil ihrer gesamten Strahlungsleistung in diesem Bereich emittieren. Figur 1 legt in dem unteren Spektrum nahe, dass der Gesamtanteil der UV-Strahlung eines Mitteldruckstrahlers in dem Bereich von 200 - 240 nm gegenüber der gesamten Emission gering ist. Hinsichtlich der biologischen Wirksamkeit in Anlagen zur Desinfektion von Wässern, die mit Mikroorganismen belastet sind, ist jedoch gerade dieser Wellenlängenbereich von größer Bedeutung. Dies kann anhand der Figur 2 nachgewiesen werden. Die Figur 2 zeigt die Wirkung von UV-Strahlung auf die DNA von verschiedenen Mikroorganismen in Abhängigkeit von der Wellenlänge. Die verschiedenen Kurven, die in Figur 2 dargestellt sind, sind auf eine relative Wirkung von 1 bei 240 nm normiert. Es ist erkennbar, dass die Wirkung von UV-Strahlung im Bereich von 240 - 300 nm für die untersuchten Mikroorganismen etwa gleich ist ( mit einer Schwankung von vielleicht etwa 50 %). Unterhalb von 240 nm ändert sich die Wirkung der UV-Strahlung auf die DNA der verschiedenen Mikroorganismen jedoch drastisch. Es gibt Mikroorganismen, die in dem kurzwelligen Bereich relativ wenig UV-Strahlung absorbieren und auf die deshalb die UV-Wirkung gering bleibt. Es gibt andererseits Mikroorganismen, die unterhalb von 240 nm einen ganz erheblichen Anstieg der UV-Absorption und damit auch der Inaktivierung durch UV-Strahlung zeigen.

Die Figur 2 zeigt also, dass die Variation der Strahlungsleistung im Bereich von 200 - 240 nm bei manchen Mikroorganismen einen erheblichen Unterschied hinsichtlich der Desinfektionsleistung einer UV-Desinfektionsanlage ausmachen kann, wenn nämlich die Wirkung der UV-Strahlung bei niedrigen Wellenlängen so stark ansteigt wie es in der Figur 2 für einige Mikroorganismen veranschaulicht ist.

Es gibt weitere Einflüsse auf das Spektrum der UV-Strahlung, die von einem Mitteldruckstrahler emittiert wird. Das Verhältnis zwischen der Strahlungsleistung im Bereich 200 - 240 nm zur Gesamtemission des Strahlers ist nur von der Bauart und der Betriebsweise des Strahlers abhängig. Wenn das UV-Licht sich von der Strahlungsquelle aus ausbreitet, wechselwirkt das Licht auch mit den verschiedenen Materialien, die in dem Strahlungsweg enthalten sind, bevor es auf die Mikroorganismen trifft. Die Materialien in Strahlungsweg sind zum einen die Gasfüllung des Strahlers selber, der Quarzkolben des Strahlers, die Luft zwischen dem Quarzkolben und einer Schutzhülle, ein eventuell zwischen dem UV-Strahler und dem zu behandelnden Wasser vorgesehenes Hüllrohr und schließlich das Wasser selbst. An diesen Materialien und ihren Oberflächen treten Absorption, Reflexion, Beugung und Streuung auf, die das Spektrum beeinflussen.

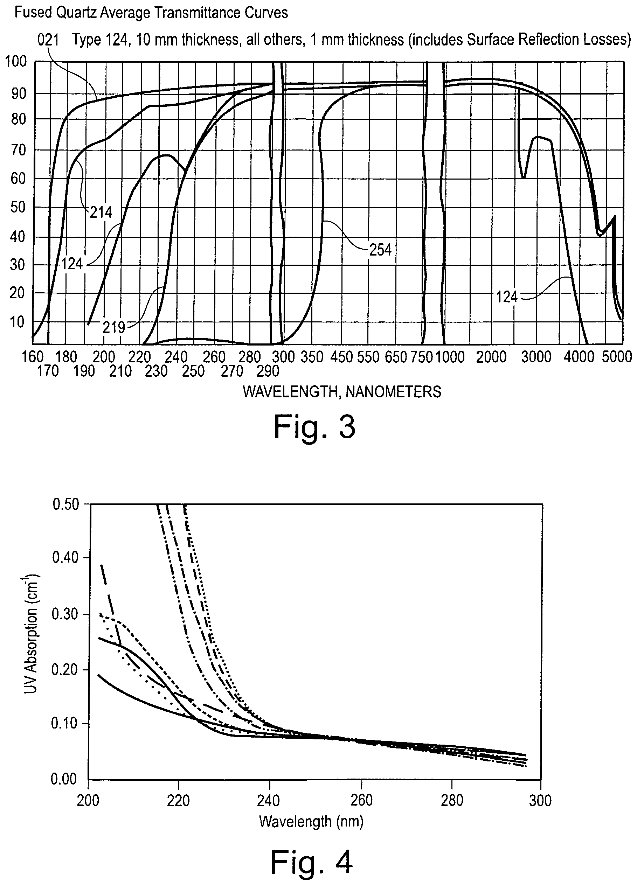

Die Figur 3 zeigt zum Beispiel die Transmissionsspektren für UV-Strahlung in verschiedenen Qualitäten von Quarzglas. In dem hier interessierenden Wellenlängenbereich von 200 - 300 nm weist das mit der Ziffer 021 bezeichnete synthetische Quarzglas die beste Transmission von durchgehend etwa 90 % auf, während das mit der Ziffer 219 bezeichnete Quarzglas bereits unterhalb von 260 nm einen deutlichen Abfall zeigt und unter 220 nm praktisch intransparent für UV-Strahlung ist. Die mit der Ziffer 124 gekennzeichnete Qualität weist ein Transmissionsminimum bei 245 nm auf und fällt ab 230 nm wiederum ab. Bei 200 nm beträgt die Transmission nur 25 %. Die Figur 3 soll veranschaulichen, dass ein Mitteldruckstrahler, der einen signifikanten Anteil seiner Strahlungsleistung im Bereich von 200 - 240 nm, also in dem Wellenlängenbereich, der nach Figur 2 für manche Mikroorganismen besonders wirkungsvoll ist, emittiert, seine Strahlungsleistung in diesem Bereich bei ungeeigneter Auswahl von Quarzglas für ein Hüllrohr seine Leistung in diesem Bereich teilweise oder vollständig einbüßen kann. Der Bereich um 260 - 300nm ist bei den verschiedenen Quarzqualitäten nahezu gleich. Wenn eine Regelung der Strahlungsleistung des Mitteldruckstrahlers auf Basis eines Sensors erfolgt, der nur im Bereich 260 - 300 nm empfindlich ist, werden Einflüsse der Absorption von verschiedenen Quarzsorten nicht berücksichtigt.

Die Figur 4 zeigt verschiedene Absorptionsspektren von Wasserproben in dem Bereich zwischen 200 und 300 nm. Während Wasserproben mit sehr geringer UV-Absorption im kurzwelligen Bereich bei 200 nm nur eine Absorption von etwa 20 % zeigen, sind Wasserproben mit höherer UV-Absorption schon ab einem Wellenlängenbereich von ca. 230 nm mit einer UV-Absorption von 40 % und mehr aufgeführt. Die Absorption bei Wellenlängen von mehr als 240 nm ist für alle Proben im Wesentlichen gleich. Auch dieses Beispiel zeigt, dass eine Regelung der Strahlungsleistung mit Sensoren, deren Empfindlichkeit zwischen 260 und 300 nm liegt, die Änderungen in der UV-Absorption des behandelnden Wassers nicht hinreichend berücksichtigen kann. Wenn erfindungsgemäß zur Steuerung und Regelung der Strahlungsleistung des Mitteldruckstrahlers ein UV-Sensor eingesetzt wird, der empfindlich ist im Bereich von 200 - 240 nm, kann die am Ort der gewünschten Wirkung eintreffende Strahlung auch bei schwankender UV-Absorption des Wasser zuverlässig geregelt werden.

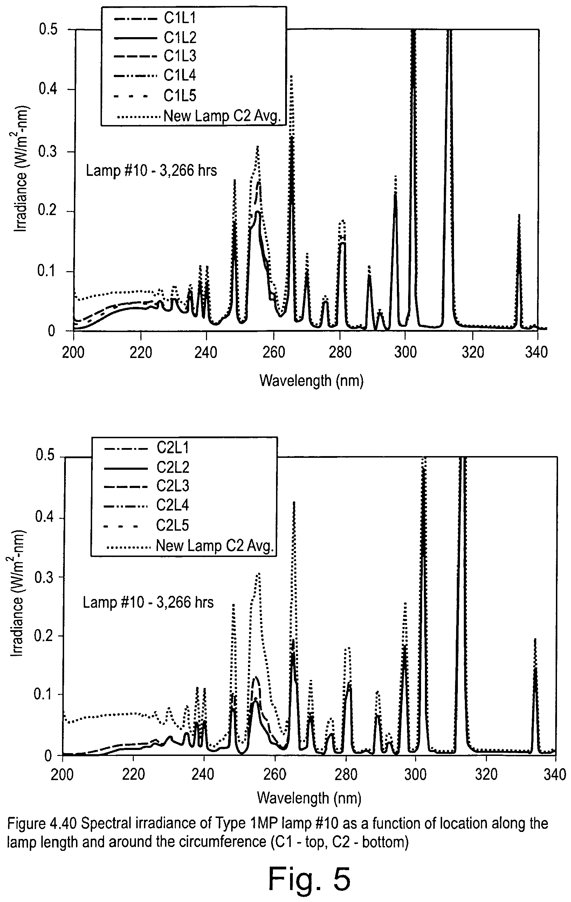

Die Figur 5 zeigt an Beispielen, wie sich die Spektren von Mitteldruckstrahlern über eine Betriebsdauer von 3.266 Stunden verändern. Die höher liegenden Emissionsspektren stellen die Leistung neuer Strahler dar, während die darunter liegenden Linien die Leistung der gealterten Strahler wiedergeben. Es ist ersichtlich, dass die Alterung die Leistung im Bereich von 260 nm um rund 50% abfallen lässt, der Leistungsabfall im Bereich von 200 - 240 nm jedoch deutlich größer ist. In dem unteren Beispiel ist der Bereich von 200 - 210 nm fast vollständig weggefallen.

Bei Regelung der Desinfektionsanlage nach der Intensität im Bereich von 260 nm würde deshalb nicht berücksichtigt, dass der Leistungsabfall in dem Bereich 200 - 240 nm wesentlich größer ist. Deshalb ist es für eine Regelung einer UV-Desinfektionsanlage vorteilhaft, nach der Strahlungsleistung im Bereich von 200 - 240 nm zu regeln.

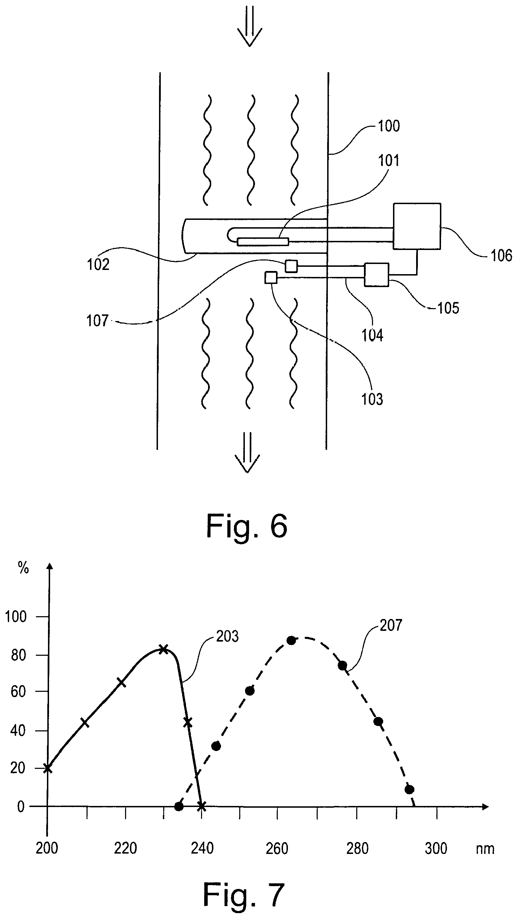

Ein Blockschaltbild einer UV-Desinfektionsanlage mit einer erfindungsgemäßen Regelung ist in der Figur 6 veranschaulicht.

Ein Kanal 100 führt einen Strom von Wasser (Abwasser oder Trinkwasser). Der Kanal kann bei Abwasserbehandlung ein offenes oder geschlossenes Gerinne sein. Bei Trinkwasserbehandlung ist üblicherweise ein geschlossener Kanal aus Edelstahl vorgesehen.

Das Wasser strömt an einem Quecksilbermitteldruckstrahler 101 vorbei, der in einem UV-transparenten Hüllrohr 102 angeordnet ist und so keinen direkten Kontakt zu dem Wasser hat.

In einem Abstand von dem Hüllrohr 102 innerhalb des Wassers ist ein erster UV-Sensor 103 angeordnet, der sensitiv für UV-Strahlung des Wellenlängenbereichs von 200 - 240 nm ist. Der Abstand des ersten UV-Sensors 103 von dem Hüllrohr 102 ist so gewählt, dass zwischen dem ersten UV-Sensor 103 und dem Hüllrohr 102 eine Strecke von Wasser liegt, wie sie im Mittel auch zwischen dem Hüllrohr 102 und den im Wasser vorhandenen Mikroorganismen liegt. Der genaue Abstand ist nicht entscheidend, es kommt vielmehr darauf an, dass so viel Wasser zwischen im Strahlungsweg zwischen dem Hüllrohr 102 und dem ersten UV-Sensor 103 liegt, dass eine Abschwächung der am ersten UV-Sensor 103 ankommenden UV-Strahlung durch die UV-Absorption des Wassers im Betrieb messbar ist.

Der erste UV-Sensor 103 gibt im Betrieb ein Signal ab, das für die eintreffende Strahlungsintensität in dem Wellenlängenbereich von 200 bis 240 nm repräsentativ ist. Das Signal wird über eine erste Signalleitung 104 an eine Regeleinheit 105 abgegeben. Die Regeleinheit 105 wiederum regelt eine Spannungsversorgung 106 so, dass der Strahler 101 die vorgesehene UV-Intensität produziert, die für die angestrebte Desinfektionsleistung erforderlich ist.

Der erste UV-Sensor 103 ist genau in dem Bereich des UV-C-Spektrums empfindlich, in dem die Desinfektionswirkung sehr stark von der biologischen Wirksamkeit und der Wellenlänge abhängt, insbesondere für verschiedene Mikroorganismen. Der erste UV-Sensor 103 empfängt die von dem Strahler 101 ausgehende Strahlung des genannten Bereich, wobei eine Änderung des Leistungsspektrums mit der Zeit, Absorption durch das Hüllrohr und das Wasser, Streuung und andere Einflüsse berücksichtigt werden. Es werden leistungsmindernde Einflüsse berücksichtigt, die sich in dem Bereich um 260 nm Wellenlänge weniger stark auswirken.

Optional kann ein weiterer Sensor als zweiter UV-Sensor 107 vorgesehen sein, der ebenfalls zur Messung der UV-Strahlung im Wasser vorgesehen ist, der jedoch wie bei den herkömmlichen Überwachungen oder Regelungen von Breitband-UV-Strahlern sein Empfindlichkeitsmaximum im längerwelligen Bereich, etwa bei 260 nm, hat. Dieser zweite UV-Sensor 107 gibt die Strahlungsintensität im längerwelligen Bereich des UV-C-Spektrums an die Regeleinheit 105 ab, die daraus dann die gesamte Strahlungsleistung im UV-C-Bereich und im kurzwelligen Teil des UV-B-Bereichs ermitteln kann, ohne jedoch den besonders wichtigen kurzwelligen Bereich des UV-C zu berücksichtigen, der von dem ersten UV-Sensor 103 erfasst wird. Aus dem Verhältnis der Intensitäten, die von dem ersten UV-Sensor 103 einerseits und dem zweiten UV-Sensor 107 andererseits gemessen werden, kann die Regeleinheit 105 auf den Zustand der Desinfektionsanlage und insbesondere des Strahlers 101 schließen und bei übermäßigem Abfall der UV-Leistung im Wellenlängenbereich des ersten UV-Sensors 103 eine Warnung erzeugen.

Die Figur 7 zeigt schließlich ein Empfindlichkeitsspektrum 203 des ersten UV-Sensors 103 und ein Empfindlichkeitsspektrum 207 des zweiten UV-Sensors 107. Die mit 203 bezeichnete Kurve für den ersten UV-Sensor 103 weist ein Empfindlichkeitsmaximum bei etwa 225 nm auf, während oberhalb von 240 nm und unterhalb von 200 nm praktisch keine Empfindlichkeit vorliegt. Die mit 207 bezeichnete Kurve des zweiten UV-Sensors 107 weist ein Maximum bei 260 nm auf, wobei unterhalb von 235 und oberhalb von 295 nm die spektrale Empfindlichkeit des zweiten UV-Sensors 107 gegen Null geht.

Die beschriebene Regelung erlaubt es also, in dem wichtigen Wellenlängenbereich von 200 nm bis 240 nm die Desinfektionsanlage genau zu überwachen und zu regeln, so dass eine genaue Information darüber vorliegt, welche Inaktivierungs- oder Desinfektionsleistung erzielt wird, und zwar ggf. auch für spezifische Mikroorganismen.

## Patentansprüche

1. Vorrichtung zur Wasserdesinfektion mit wenigstens einem in einem Kanal (100) angeordneten Breitband-UV-Strahler (101), und wenigstens einem ersten UV-Sensor (103), der in dem Wasser in einem Abstand von dem Breitband-UV-Strahler (101) angeordnet ist, und wobei der erste UV-Sensor (103) mit einer Regeleinheit (105) verbunden ist, die zur Regelung der Leistung des Breitband-UV-Strahlers (101) oder des Volumenstroms an Wasser durch den Kanal (100) eingerichtet ist, **dadurch gekennzeichnet, dass** der erste UV-Sensor (103) ein Empfindlichkeitsmaximum für UV-Strahlung in einem Wellenlängenbereich zwischen 200 nm und 230 nm aufweist.

2. Vorrichtung nach Anspruch 1, **dadurch gekennzeichnet, dass** der erste UV-Sensor (103) ein Empfindlichkeitsmaximum für UV-Strahlung bei 220 nm aufweist.

3. Vorrichtung nach einem der vorhergehenden Ansprüche, **d**a**durch gekennzeichnet**, dass ein zweiter UV-Sensor (107) vorgesehen ist, wobei der zweite UV-Sensor (107) ein Empfindlichkeitsmaximum zwischen 240 nm und 300 nm, vorzugsweise bei 260 nm aufweist.

4. Verfahren zur Überwachung und Steuerung einer Wasserdesinfektionsanlage mit wenigstens einem in einem Kanal (100) angeordneten Breitband-UV-Strahler (101), wobei wenigstens ein erster UV-Sensor (103) vorgesehen ist, der in dem Wasser in einem Abstand von dem Breitband-UV-Strahler (101) angeordnet ist, und wobei der erste UV-Sensor (103) mit einer Regeleinheit (105) verbunden ist, die zur Regelung der Leistung des Breitband-UV-Strahlers (101) oder des Volumenstroms an Wasser durch den Kanal (100) eingerichtet ist, **dadurch gekennzeichnet, dass**
- der erste UV-Sensor (103) ein Empfindlichkeitsmaximum im Bereich zwischen 200 nm und 230 nm aufweist,
- ein Signal des ersten UV-Sensors (103) im Betrieb ausgewertet wird,
- aus dem Signal des ersten UV-Sensors (103) eine UV-Dosis in dem Wellenlängenbereich von 200 nm bis 240 nm berechnet wird, und
- die am Ort des ersten UV-Sensors (103) ermittelte UV-Dosis als Maß für die Berechnung der Desinfektionsleistung der Wasserdesinfektionsanlage verwendet wird.

5. Verfahren nach Anspruch 4, **dadurch gekennzeichnet, dass** für die Berechnung der Desinfektionsleistung ein UV-Wirkungsspektrum eines vorbestimmten Mikroorganismus zugrunde gelegt wird.

6. Verfahren nach Anspruch 5, **dadurch gekennzeichnet, dass** für die Berechnung der Desinfektionsleistung der Mikroorganismus aus einer Gruppe auswählbar ist, die Viren, Bakterien und Einzeller umfasst.

7. Verfahren nach einem der vorhergehenden Ansprüche 4 bis 6, **dadurch gekennzeichnet, dass** ein zweiter UV-Sensor (107) mit einem Empfindlichkeitsmaximum zwischen 240 nm und 300 nm vorgesehen ist, und dass aus dem Verhältnis der Signale des ersten UV-Sensors (103) und des zweiten UV-Sensors (107) ein Messwert ermittelt wird, der die Dosisberechnung unter Berücksichtigung der Alterung des Breitband-UV-Strahlers (101) und der spektralen Absorption des Wasser ermöglicht.

8. Verfahren nach einem der vorhergehenden Ansprüche 4 bis 7, **dadurch gekennzeichnet , dass** in Abhängigkeit von dem Signal des ersten UV-Sensors (103) die elektrische Leistung des Breitband-UV-Strahlers (101) geregelt wird.

## Claims

1. Device for water disinfection, having at least one broadband UV radiator (101) arranged in a channel (100), and at least one first UV sensor (103) which is arranged in the water at a distance from the broadband UV radiator, wherein the first UV sensor (103) is connected to a regulating unit (105) which is configured in order to regulate the power of the broadband UV radiator (101) or the volume flow rate of water through the channel, **characterised in that** the first UV sensor (103) has a sensitivity maximum for UV radiation in a wavelength range of between 200 nm and 230 nm.

2. Device according to Claim 1, **characterised in that** the first UV sensor (103) has a sensitivity maximum for UV radiation at 220 nm.

3. Device according to any one of the preceding claims, **characterised in that** a second UV sensor (107) is provided, the second UV sensor (107) having a sensitivity maximum between 240 nm and 300 nm, preferably at 260 nm.

4. Method for monitoring and controlling a water disinfection plant having at least one broadband UV radiator (101) arranged in a channel (100), wherein at least one first UV sensor (103) is provided, which is arranged in the water at a distance from the broadband UV radiator, and wherein the first UV sensor (103) is connected to a regulating unit (105) which is configured in order to regulate the power of the broadband UV radiator (101) or the volume flow rate of water through the channel, **characterised in that**
- the first UV sensor (103) has a sensitivity maximum in a wavelength range of between 200 nm and 230 nm,
- a signal of the first UV sensor (103) is evaluated during operation,
- a UV dose in the wavelength range of from 200 nm to 240 nm is calculated from the signal of the first UV sensor (103), and
- the UV dose determined at the position of the first UV sensor (103) it is used as a measure for calculating the disinfection power of the water disinfection plant.

5. Method according to Claim 4, **characterised in that** a UV action spectrum of a predetermined microorganism is used as the basis for the calculation of the disinfection power.

6. Method according to Claim 5, **characterised in that,** for the calculation of the disinfection power, the microorganism can be selected from a group which comprises viruses, bacteria and unicellular organisms.

7. Method according to any one of the preceding Claims 4 to 6, **characterised in that** a second UV sensor (107) having a sensitivity maximum between 240 nm and 300 nm is provided, and **in that** a measurement value that allows dose calculation while taking into account the ageing of the broadband UV radiator (101) and the spectral absorption of the water is determined from the ratio of the signals of the first UV sensor (103) and of the second UV sensor (107).

8. Method according to any one of the preceding Claims 4 to 7, **characterised in that** the Electrical power of the broadband UV radiator (101) is regulated as a function of the signal of the first UV sensor (103).

## Revendications

1. Procédé de désinfection d'eau comprenant au moins un élément d'irradiation UV à spectre large (101) disposé dans un canal (100) et au moins un premier capteur UV (103), lequel est disposé dans l'eau à distance de l'élément d'irradiation UV à spectre large (101) et où le premier capteur UV (103) est relié à une unité de réglage (105) qui est destinée à régler la puissance de l'élément d'irradiation UV à spectre large (101) ou le débit volumique d'eau circulant dans le canal (100), **caractérisé en ce que** le premier capteur UV (103) présente un maximum de sensibilité au rayonnement UV dans une gamme des longueurs d'ondes comprise entre 200 nm et 230 nm.

2. Procédé selon la revendication 1, **caractérisé en ce que** le premier capteur UV (103) présente un maximum de sensibilité au rayonnement UV lors de 220 nm.

3. Procédé selon l'une des revendications précédentes, **caractérisé en ce que** l'on prévoit un deuxième capteur UV (107), où le deuxième capteur UV (107) présente un maximum de sensibilité entre 240 nm et 300 nm, de préférence lors de 260 nm.

4. Dispositif destiné à surveiller et à commander une installation de désinfection d'eau, comprenant au moins un élément d'irradiation UV à spectre large (101) disposé dans un canal (100), où l'on prévoit au moins un premier capteur UV (103) qui est disposé dans l'eau à distance de l'élément d'irradiation UV à spectre large (101), et où le premier capteur UV (103) est relié à une unité de réglage (105) qui est destinée à régler la puissance de l'élément d'irradiation UV à spectre large (101) ou du débit volumique d'eau à travers le canal (100), **caractérisé en ce que**
- le premier capteur UV (103) présente un maximum de sensibilité dans une gamme entre 200 nm et 230 nm,
- un signal du premier capteur UV (103) est évalué en fonctionnement,
- l'on calcule, à partir du signal du premier capteur UV (103), une dose d'UV dans la gamme des longueurs d'ondes de 200 nm jusqu'à 240 nm, et
- la dose d'UV déterminée sur le lieu du premier capteur UV (103) est utilisée en tant que mesure pour le calcul de la puissance de désinfection de l'installation de désinfection d'eau.

5. Procédé selon la revendication 4, **caractérisé en ce que** l'on prend comme base un spectre d'action UV d'un micro-organisme prédéterminé pour le calcul de la puissance de désinfection.

6. Procédé selon la revendication 5, **caractérisé en ce que,** pour le calcul de la puissance de désinfection, le micro-organisme peut être sélectionné à partir d'un groupe qui comporte des virus, bactéries et organismes unicellulaires.

7. Procédé selon l'une des revendications précédentes de 4 à 6, **caractérisé en ce que** l'on prévoit un deuxième capteur UV (107) avec un maximum de sensibilité entre 240 nm et 300 nm, et que l'on détermine une valeur de mesure à partir du rapport des signaux du premier capteur UV (103) et du deuxième capteur UV (107), cette valeur de mesure permettant le calcul de dose en tenant compte du vieillissement de l'élément d'irradiation UV à spectre large (101) et de l'absorption spectrale de l'eau.

8. Procédé selon l'une des revendications précédentes de 4 à 7, **caractérisé en ce que** la puissance électrique de l'élément d'irradiation UV à spectre large (101) est réglée en fonction du signal du premier capteur UV (103).
